# EUROPEAN PATENT SPECIFICATION

(11) **EP 4 289 333 B1**
(45) Date of publication and mention of the grant of the patent: **14.01.2026**
(21) Application number: 23174044.0
(22) Date of filing: 17.05.2023
(51) Int. Cl.: A61B 1/00

(54) **ENDOSCOPE DEVICE**
ENDOSKOPVORRICHTUNG
DISPOSITIF D'ENDOSCOPE

(30) Priority: 09.06.2022 JP 2022093823
(43) Date of publication of application: 13.12.2023
(73) Proprietor: FUJIFILM Corporation, Tokyo 106-8620 (JP)
(72) Inventor: MATSUSHITA, Motohiko, Kanagawa, 258-8538 (JP)
(74) Representative: Dehns Germany Partnerschaft mbB

(56) References cited:
- JP-A- 2005 007 030
- US-A1- 2006 116 586
- US-A1- 2007 055 101
- US-A1- 2016 220 097

## Description

### BACKGROUND OF THE INVENTION

### 1. Field of the Invention

The present invention relates to an endoscope device that controls inflation and deflation of a balloon of an endoscope.

### 2. Description of the Related Art

In the related art, in a medical field, a procedure of inserting an insertion part of an endoscope into an intestinal tract (lumen) such as a large intestine and a small intestine to observe, diagnose, and treat an inner wall surface of the intestinal tract has been performed. Since the intestinal tract is complicatedly bent, it is difficult to transmit force to a distal end of the insertion part simply by pushing the insertion part of the endoscope, and it is difficult to insert the endoscope into a deep portion.

Therefore, there is known a double-balloon type endoscope device in which balloons capable of inflating and deflating are respectively attached to the insertion part of the endoscope and a distal end portion of an overtube covering the insertion part (see JP2016-137206A, JP2005-296258A, JP2006-149999A, JP2007-111542A, and JP2005-261781A). With this endoscope device, inflation and deflation of each balloon can be individually controlled by supplying and sucking air from a balloon controller to an inside of each balloon. This allows the insertion part to be inserted into an inner side (deep portion) of the complicatedly bent intestinal tract by alternately inserting the insertion part and the overtube while temporarily fixing each balloon individually and at a predetermined time to the intestinal tract.

In the endoscope device disclosed in JP2016-137206A, JP2005-296258A, JP2006-149999A, and JP2007-111542A, switching between inflation and deflation of each balloon can be performed individually by operating a remote controller connected to the balloon controller by an operator (including an assistant, the same applies hereinafter). On the other hand, a switch (controller operation switch) connected to the balloon controller is provided in an operating part of the endoscope of the endoscope device disclosed in JP2005-261781A. By operating this switch by the operator, switching between inflation and deflation of each balloon can be performed individually.

In addition, in the endoscope device disclosed in JP2006-149999A and JP2007-111542A, a balloon state image showing whether each balloon is in an inflated state or a deflated state is displayed on a monitor. US 2007/0055101 A1 relates to an endoscope balloon control device.

### SUMMARY OF THE INVENTION

Incidentally, in a case in which the switch provided in the operating part of the endoscope is operated to switch between inflation and deflation of each balloon as in the endoscope device disclosed in JP2005-261781A, there is a problem that it is difficult to instantly grasp which operation is being performed because the operator gazes at the monitor.

In addition, even in a case in which the balloon state image of each balloon is displayed on the monitor as in the endoscope device disclosed in JP2006-149999A and JP2007-111542A, it is premised that a switching operation between inflation and deflation of the balloon is performed using the remote controller, an operation panel, or the like. Therefore, the operator who operates the switch of the operating part cannot directly grasp which operation is being performed.

The present invention has been made in view of such circumstances, and an object of the present invention is to provide an endoscope device that enables an operator to easily grasp which operation is being operated in a case in which the operator operates a controller operation switch provided in an endoscope.

An endoscope device is provided as defined by claim 1.

With the endoscope device, an operator can easily grasp what kind of balloon operation has been performed on the controller operation switch even in a state in which the operator gazes at the monitor.

In the endoscope device according to an embodiment of the present invention, the remote controller includes a plurality of types of individual operation switches corresponding to the plurality of types of balloon operations, the remote controller image includes images of the plurality of types of individual operation switches, and the processor is configured to, in a case in which the balloon operation is input to the controller operation switch, change a display mode of an image of the individual operation switch corresponding to the type of the balloon operation. Accordingly, it is possible to easily grasp which operation the operator is performing in a case in which the operator performs the balloon operation using the controller operation switch.

In the endoscope device according to an embodiment of the present invention, the balloon is provided at at least one of a distal end portion of an insertion part of the endoscope or a distal end portion of an overtube through which the insertion part is inserted.

In the endoscope device according to an embodiment of the present invention, the balloon is provided at the distal end portion of the insertion part and the distal end portion of the overtube, the balloon controller controls inflation and deflation of the balloon for each of the balloons, the remote controller is capable of inputting the balloon operation for each of the balloons, and the processor is configured to reflect the type of the balloon operation input to the controller operation switch on the remote controller image, for each of the balloons. Accordingly, even with a double-balloon type endoscope device, in a case in which the operator performs the balloon operation using the controller operation switch, it is possible to easily grasp which operation the operator is performing.

In the endoscope device according to an embodiment of the present invention, the endoscope device further comprises: an emergency stop switch for bringing the balloon controller to an emergency stop, in which the processor is configured to, in a case in which the emergency stop switch is operated, cause the monitor to display information indicating that the balloon controller has been brought to an emergency stop. Accordingly, it is possible to notify the operator that the balloon controller has been brought to an emergency stop.

In the endoscope device according to an embodiment of the present invention, the emergency stop switch is a foot switch connected to the balloon controller. Accordingly, even in a case in which both hands of the operator are full, it is possible to quickly perform an emergency stop operation of the balloon controller.

In the endoscope device according to an embodiment of the present invention, the endoscope device further comprises: a pressure detection sensor that repeatedly detects a pressure inside the balloon, in which the processor is configured to cause the monitor to display information indicating the pressure each time the pressure detection sensor detects the pressure. Accordingly, it is possible to easily grasp the pressure in the balloon in a state in which the operator gazes at the monitor.

In the endoscope device according to an embodiment of the present invention, the balloon is provided at a distal end portion of an insertion part of the endoscope and a distal end portion of an overtube through which the insertion part is inserted, the pressure detection sensor repeatedly detects the pressure for each of the balloons, and the processor is configured to cause the monitor to display the information indicating the pressure for each of the balloons. Accordingly, it is possible to easily grasp the pressure in each balloon in a state in which the operator gazes at the monitor.

In the endoscope device according to an embodiment of the present invention, the controller operation switch is provided in an operating part provided on a base end side of an insertion part of the endoscope.

In the endoscope device according to an embodiment of the present invention, the remote controller includes a plurality of types of individual operation switches corresponding to the plurality of types of balloon operations, the operating part is provided with a plurality of multifunctional switches to which a plurality of types of operations are capable of being selectively assigned, and the controller operation switch is the plurality of multifunctional switches to which functions of the plurality of types of individual operation switches are individually assigned. Accordingly, the present invention can be applied to an existing endoscope device comprising a multifunctional switch.

According to the present invention, it is possible to easily grasp which operation an operator is performing in a case in which the operator operates a controller operation switch provided in an endoscope.

### BRIEF DESCRIPTION OF THE DRAWINGS

Fig. 1 is a configuration diagram of a double-balloon type endoscope device according to a first embodiment.
Fig. 2 is a pipe line diagram of an endoscope device.
Fig. 3 is a front view of a remote controller.
Figs. 4A to 4D are explanatory diagrams for describing a display pattern of a first balloon state display part and a second balloon state display part of the remote controller.
Fig. 5 is an explanatory diagram for describing assignment of functions of some switches of the remote controller to multifunctional switches.
Fig. 6 is an explanatory diagram showing an example of an endoscopy screen displayed on a monitor in a balloon operation presentation mode by a processor device.
Fig. 7 is an enlarged view of a remote controller image in a second display region.
Figs. 8A to 8D are explanatory diagrams for describing an example of changing a display mode of an inflated state display part image and a deflated state display part image in accordance with an inflation operation or a deflation operation with respect to the multifunctional switches.
Fig. 9 is an explanatory diagram showing an example of an endoscopy screen displayed on a monitor by a processor device of an endoscope device according to a second embodiment.
Fig. 10 is an enlarged view of a remote controller image, first pressure information, and second pressure information according to the second embodiment.
Fig. 11 is a configuration diagram of an endoscope device according to a third embodiment.
Fig. 12 is an explanatory diagram for describing a display of warning information by a monitor.

### DESCRIPTION OF THE PREFERRED EMBODIMENTS

### First Embodiment

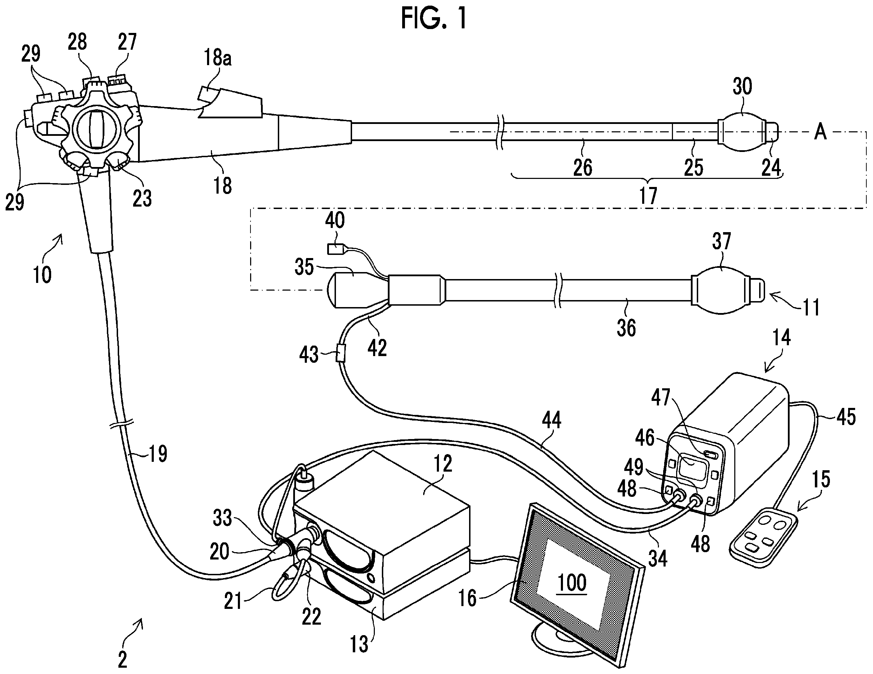

Fig. 1 is a configuration diagram of a double-balloon type endoscope device 2 according to a first embodiment. As shown in Fig. 1, an endoscope device 2 comprises an endoscope 10 which is an electronic endoscope with a balloon, an overtube 11 with a balloon, a light source device 12, a processor device 13, a balloon controller 14, a remote controller 15, and a monitor 16.

The endoscope 10 comprises a flexible insertion part 17 and an operating part 18 provided consecutively to a base end side of the insertion part 17.

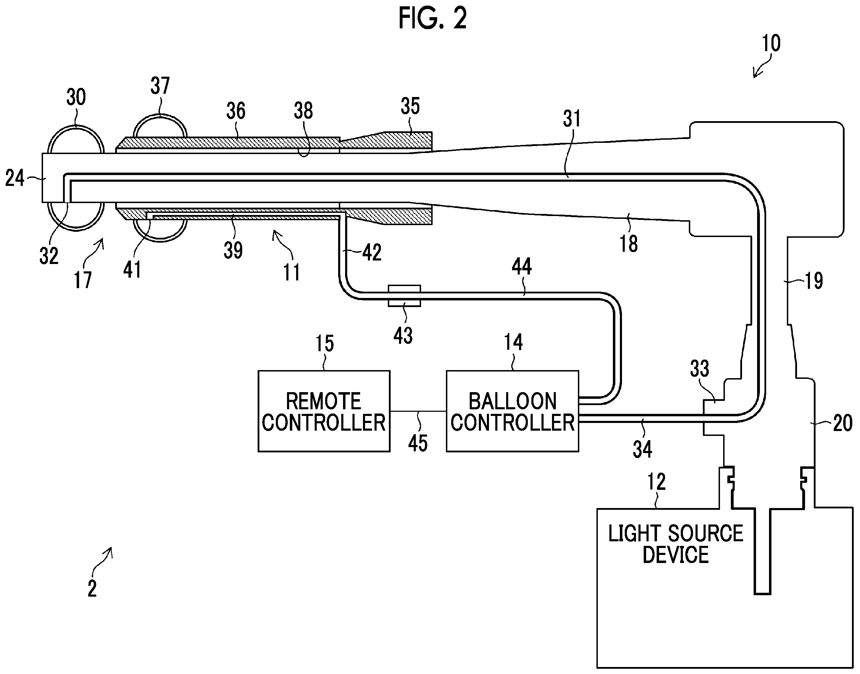

The operating part 18 is used for an operator to perform an operation, and is also referred to as a hand operating part. A universal cable 19 is connected to the operating part 18. The universal cable 19 includes a signal cable (not shown), a light guide (not shown), a first supply/discharge pipe line 31 (see Fig. 2), and the like. A light source connector 20 is provided at a distal end of the universal cable 19.

A cable 21 is branched from the light source connector 20, and the processor connector 22 is provided at a distal end the cable 21. The light source connector 20 is attachably and detachably connected to the light source device 12, and the processor connector 22 is attachably and detachably connected to the processor device 13.

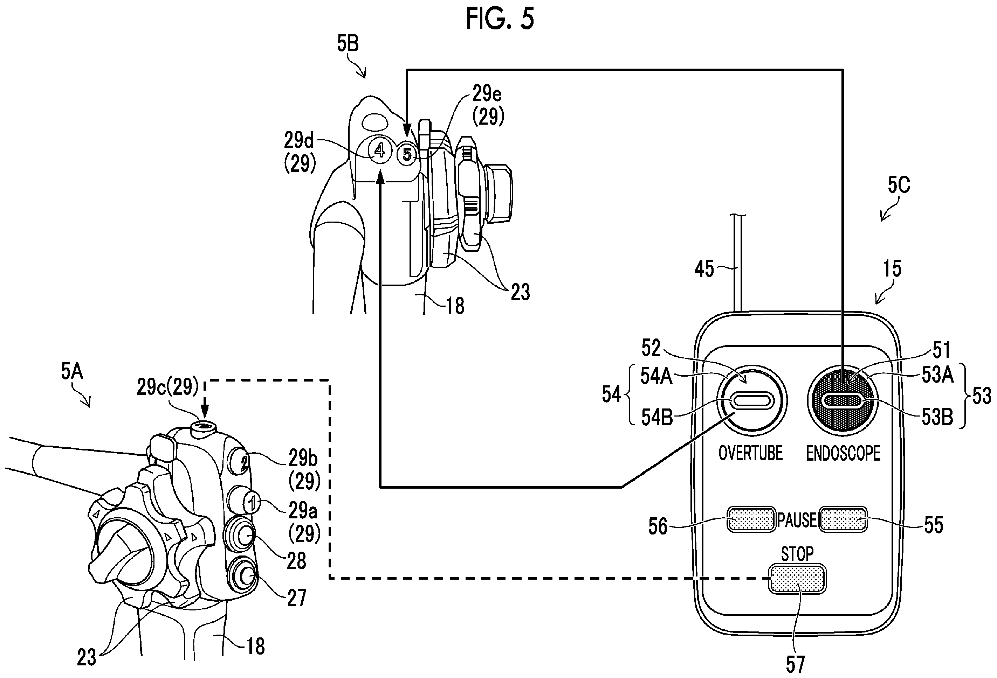

The operating part 18 is provided with a plurality of multifunctional switches 29 in addition to a pair of known angle knobs 23, an air supply/water supply button 27, a suction button 28, and a treatment tool insertion part 18a. Each of the multifunctional switches 29, which will be described in detail below, can selectively assign a plurality of types of operations of the endoscope device 2, such as imaging of an area to be observed, switching of special light observation, and inflation and deflation of the respective balloons 30 and 37, which will be described below (see Fig. 5). The term "switch" in the present specification includes various known operating parts and operating members such as a button type, a lever type, and a touch sensor type.

The insertion part 17 is inserted into a lumen (for example, a small intestine or a large intestine) of a subject. The insertion part 17 comprises a distal end portion 24, a bendable portion 25, and a soft portion 26 from a distal end side to a base end side.

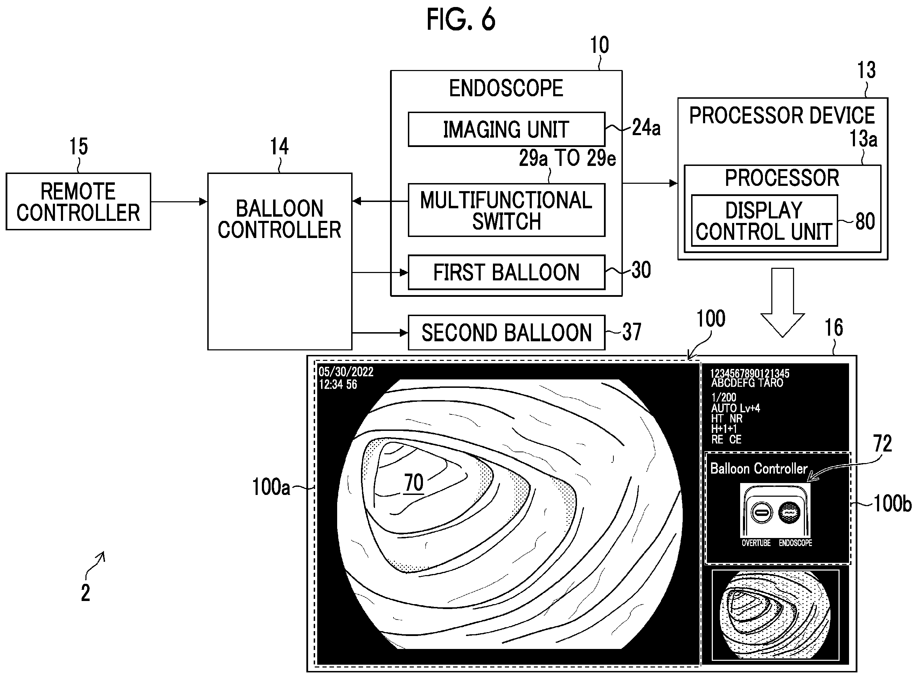

**In** addition to an imaging unit 24a (see Fig. 6) that captures an image of an area to be observed in the lumen, the distal end portion 24 comprises a known observation window, an illumination window for emitting illumination light supplied from the light source device 12, a nozzle for air and water supplying, and a forceps port communicating with the treatment tool insertion part 18a via a treatment tool channel (not shown) in the insertion part 17, although not shown. The imaging unit 24a is provided behind the observation window, and captures an observation image of the area to be observed through the observation window. **In** addition, a signal cable (not shown) is connected to the imaging unit 24a, and this signal cable is connected to the processor device 13 via the aforementioned insertion part 17, operating part 18, universal cable 19, light source connector 20, cable 21, processor connector 22, and the like. Accordingly, an imaging signal of the area to be observed imaged by the imaging unit 24a is output to the processor device 13 via the signal cable.

The bendable portion 25 is remotely bent (angle operation) by rotating the pair of angle knobs 23 provided in the operating part 18. Accordingly, a distal end surface (observation window and illumination window) of the distal end portion 24 can be directed in a desired direction.

The soft portion 26 is provided consecutively to a base end of the bendable portion 25 and has flexibility. The soft portion 26 has a length of several meters to make the distal end portion 24 reach a target position in the body.

A first balloon 30 corresponding to the balloon of the present invention is attachably and detachably attached to the distal end portion 24. The first balloon 30 is formed in a substantially tubular shape of which an end portion is narrowed by an elastic material such as rubber, and has a balloon distal end portion and a balloon base end portion each having a small diameter, and a central swollen portion. The distal end portion 24 is inserted into the first balloon 30 such that the first balloon 30 is located at a predetermined position of the distal end portion 24, and then, for example, a rubber ring is fitted into the balloon distal end portion and the balloon base end portion. Thus, the first balloon 30 is fixed to the distal end portion 24.

The overtube 11 attachably and detachably covers the insertion part 17. The overtube 11 comprises a grip portion 35 gripped by the operator, a main body portion 36, and a second balloon 37 corresponding to the balloon of the present invention.

The grip portion 35 is a tubular body made of a hard material such as plastic. The main body portion 36 is formed in a substantially tubular shape by a flexible material such as polyurethane, and is externally fitted and fixed to a distal end side of the grip portion 35.

The second balloon 37 is formed in a substantially tubular shape of which an end portion is narrowed by an elastic material such as rubber, and has a balloon distal end portion and a balloon base end portion each having a small diameter, and a central swollen portion. The second balloon 37 covers an outer peripheral surface of a distal end of the main body portion 36, and is fixed to the main body portion 36 by, for example, winding a thread around the balloon distal end portion and the balloon base end portion and applying an adhesive thereon.

Fig. 2 is a pipe line diagram of the endoscope device 2. In Fig. 2, the endoscope 10 and the overtube 11 are shown in a simplified manner.

As shown in Fig. 2 and Fig. 1 described above, the endoscope 10 is provided with a first supply/discharge pipe line 31 for supplying and sucking air into the inside of the first balloon 30. The first supply/discharge pipe line 31 is a flexible tube, and is inserted into the insertion part 17, the operating part 18, the universal cable 19, and the light source connector 20.

A distal end side of the first supply/discharge pipe line 31 communicates with an opening 32 for a balloon formed on an outer peripheral surface of the distal end portion 24. The opening 32 is formed at an attaching/detaching position of the first balloon 30 on the outer peripheral surface of the distal end portion 24. In addition, an endoscope-side cap 33 is provided on a base end side of the first supply/discharge pipe line 31.

The endoscope-side cap 33 is formed integrally with the light source connector 20. The endoscope-side cap 33 is connected to the balloon controller 14 via a tube 34. Accordingly, by the balloon controller 14 described below, air can be supplied to the inside of the first balloon 30 to inflate the first balloon 30 or, conversely, air can be sucked from the inside of the first balloon 30 to deflate the first balloon 30, via the tube 34, the endoscope-side cap 33, the first supply/discharge pipe line 31, and the opening 32. Note that the term "air" as used herein is gas for inflating the first balloon 30 (also including the second balloon 37 described below), and a type (component) thereof is not particularly limited. In addition, various fluids including a liquid such as water may be used instead of "air".

A supply pipe line 38 and a second supply/discharge pipe line 39 are formed inside the main body portion 36 of the overtube 11 in an axial direction thereof. The supply pipe line 38 is a hole through which the insertion part 17 of the endoscope 10 is inserted, and an inner diameter thereof is formed to be slightly larger than an outer diameter of the insertion part 17.

In a case in which the overtube 11 is used, a lubricant such as water is supplied to an inner peripheral surface (gap between the insertion part 17 and the main body portion 36) of the supply pipe line 38 to reduce friction between the insertion part 17 and the main body portion 36. The lubricant is injected from a connector 40 (see Fig. 1) communicating with the supply pipe line 38 by a syringe (not shown) or the like.

The second supply/discharge pipe line 39 is a pipe line for supplying and sucking air to the second balloon 37, and is provided in a pipe wall of the supply pipe line 38. A distal end side of the second supply/discharge pipe line 39 communicates with an opening 41 for a balloon formed on an outer peripheral surface of the main body portion 36. The opening 41 is formed at an attaching/detaching position of the second balloon 37 on the outer peripheral surface of the main body portion 36. In addition, a tube 42 having a small diameter is provided consecutively to a base end side of the second supply/discharge pipe line 39, and a connector 43 is provided consecutively to a base end side of the tube 42.

A tube 44 is connected to the connector 43, and the tube 44 is further connected to the balloon controller 14. Accordingly, by the balloon controller 14 described below, air can be supplied to the inside of the second balloon 37 to inflate the second balloon 37 or, conversely, air can be sucked from the inside of the second balloon 37 to deflate the second balloon 37, via the tube 44, the connector 43, the tube 42, the second supply/discharge pipe line 39, and the opening 41.

Returning to Fig. 1, the balloon controller 14 supplies and sucks air to and from the respective balloons 30 and 37 independently in order to alternately inflate and deflate the first balloon 30 of the endoscope 10 and the second balloon 37 of the overtube 11. The balloon controller 14 is provided with a pump, a solenoid valve, and the like. In addition, a remote controller 15 is electrically connected to the balloon controller 14 via a cable 45.

Further, some of a plurality of multifunctional switches 29 provided in the operating part 18 are electrically connected to the balloon controller 14 via a signal cable (not shown).

The balloon controller 14 individually controls the inflation and deflation of the respective balloons 30 and 37 in accordance with an operation input with respect to the remote controller 15 or the multifunctional switch 29 by the operator. For example, the balloon controller 14 individually supplies air to the respective balloons 30 and 37 to individually inflate the respective balloons 30 and 37, or controls the air pressure to a certain value to individually maintain the respective balloons 30 and 37 in an inflated state. In addition, the balloon controller 14 individually sucks air from the respective balloons 30 and 37 to individually deflate the respective balloons 30 and 37, or controls the air pressure to a certain value to individually maintain the respective balloons 30 and 37 in a deflated state.

A display part 46, a power switch 47, an emergency stop switch 48, a tube connecting portion 49, and the like are provided on a front surface of the balloon controller 14.

In a case in which the respective balloons 30 and 37 are inflated or deflated, the display part 46 displays a pressure value of the balloons 30 and 37 and information indicating an inflation and deflation state (whether the inflated state or the deflated state) of the respective balloons 30 and 37. In addition, an error code is displayed on the display part 46 in a case in which an abnormality, such as tear in the respective balloons 30 or 37, occurs. In a case in which the error code is displayed on the display part 46, the balloon controller 14 outputs a buzzer sound from a speaker (not shown).

The power switch 47 is used to turn on and off the power of the balloon controller 14. The emergency stop switch 48 is used for an emergency stop of the balloon controller 14 (an emergency stop of the inflation and deflation operation of the respective balloons 30 and 37). The emergency stop referred to here includes, for example, stopping the inflation and deflation operation of the respective balloons 30 and 37, or deflating the respective balloons 30 and 37. Note that the emergency stop may be executed individually for the respective balloons 30 and 37.

The aforementioned tubes 34 and 44 are each connected to the tube connecting portion 49. Although not shown, the tube connecting portion 49 is provided with a backflow prevention unit. The backflow prevention unit prevents a body fluid or the like from flowing into the balloon controller 14 in a case in which the respective balloons 30 and 37 are torn.

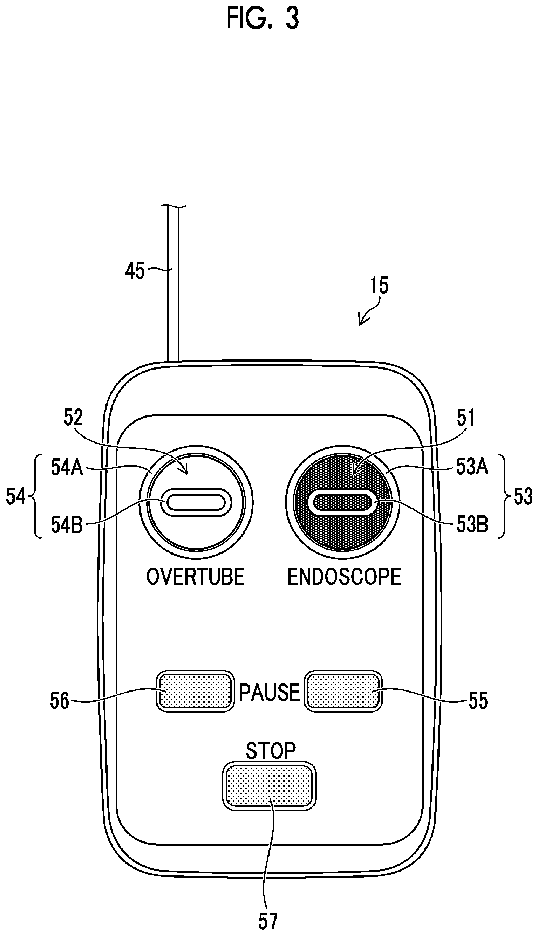

Fig. 3 is a front view of the remote controller 15. As shown in Fig. 3, the remote controller 15 is used for the input of a plurality of types of balloon operations including an inflation operation and a deflation operation of the respective balloons 30 and 37.

The remote controller 15 has a shape appropriate for being gripped by the operator, and is provided with, on a front surface, a first balloon operating part 51, a second balloon operating part 52, a first balloon state display part 53, a second balloon state display part 54, a first balloon pause button 55, a second balloon pause button 56, a stop button 57, and the like. The first balloon operating part 51, the second balloon operating part 52, the first balloon pause button 55, the second balloon pause button 56, and the stop button 57 are used for the plurality of types of balloon operations described above, and correspond to a plurality of types of individual operation switches of the present invention.

The first balloon operating part 51 is positioned on a right side of the remote controller 15 in a case in which the remote controller 15 is viewed from the front, and is a circular toggle switch for performing the inflation operation and the deflation operation of the first balloon 30 with respect to the balloon controller 14. In the present embodiment, the first balloon operating part 51 is also formed to be black (indicated by hatching in the figure) in accordance with the general color (black) of the endoscope 10.

Each time the first balloon operating part 51 is pushed, the inflation operation of the first balloon 30 and the deflation operation of the first balloon 30 are alternately input to the balloon controller 14. Accordingly, in a case in which the inflation operation of the first balloon 30 is input, the balloon controller 14 supplies air to the first balloon 30 to inflate the first balloon 30, and, conversely, in a case in which the deflation operation of the first balloon 30 is input, the balloon controller 14 sucks air from the first balloon 30 to deflate the first balloon 30.

The first balloon state display part 53 is provided in the first balloon operating part 51, and has an inflated state display part 53A and a deflated state display part 53B. The inflated state display part 53A displays an inflated state in which the first balloon 30 is inflated by supplying air from the balloon controller 14 to the first balloon 30. The deflated state display part 53B displays a deflated state in which the first balloon 30 is deflated by sucking air from the first balloon 30.

The inflated state display part 53A is disposed along an outer periphery of the first balloon operating part 51, and is a ring-shaped state display part that indicates an inflated state of the first balloon 30. The deflated state display part 53B is disposed inside the inflated state display part 53A (on the first balloon operating part 51), and is a flat-shape state display part that indicates a deflated state of the first balloon 30. Since the detailed configuration of the inflated state display part 53A and the deflated state display part 53B is a known technology (see JP2016-137206A above), specific description thereof will be omitted here.

The inflated state display part 53A switches to a display state (light emitting state) in a case in which the first balloon 30 is in an inflated state, and switches to a non-display state (non-light emitting state) in a case in which the first balloon 30 is in a deflated state. On the other hand, the deflated state display part 53B switches to a display state (light emitting state) in a case in which the first balloon 30 is in a deflated state, and switches to a non-display state (non-light emitting state) in a case in which the first balloon 30 is in an inflated state. Therefore, any one of the inflated state display part 53A or the deflated state display part 53B is in a display state, and the other is in a non-display state.

The second balloon operating part 52 is provided on a left side of the remote controller 15 in a case in which the remote controller 15 is viewed from the front, and located at a position symmetrical to the first balloon operating part 51 with respect to a center line of the remote controller 15. The second balloon operating part 52 is a circular toggle switch for performing the inflation operation and the deflation operation of the second balloon 37 with respect to the balloon controller 14. In the present embodiment, the second balloon operating part 52 is also formed to be white in accordance with the general color (white) of the overtube 11.

Each time the second balloon operating part 52 is pushed, the inflation operation of the second balloon 37 and the deflation operation of the second balloon 37 are alternately input to the balloon controller 14. Accordingly, in a case in which the inflation operation of the second balloon 37 is input, the balloon controller 14 supplies air to the second balloon 37 to inflate the second balloon 37, and, conversely, in a case in which the deflation operation of the second balloon 37 is input, the balloon controller 14 sucks air from the second balloon 37 to deflate the second balloon 37.

The second balloon state display part 54 has an inflated state display part 54A and a deflated state display part 54B. The inflated state display part 54A is provided along an outer periphery of the second balloon operating part 52, and is a ring-shaped state display part that indicates an inflated state in which the second balloon 37 is inflated. The deflated state display part 54B is provided inside the inflated state display part 54A (on the second balloon operating part 52), and is a flat-shape state display part that indicates a deflated state in which the second balloon 37 is deflated.

The inflated state display part 54A switches to a display state (light emitting state) in a case in which the second balloon 37 is in an inflated state, and switches to a non-display state (non-light emitting state) in a case in which the second balloon 37 is in a deflated state. On the other hand, the deflated state display part 54B switches to a display state (light emitting state) in a case in which the second balloon 37 is in a deflated state, and switches to a non-display state (non-light emitting state) in a case in which the second balloon 37 is in an inflated state. Therefore, any one of the inflated state display part 54A or the deflated state display part 54B is in a display state, and the other is in a non-display state.

In this way, by referring to the display of the inflated state display part 53A and the deflated state display part 53B and the display of the inflated state display part 54A and the deflated state display part 54B, the operator can discriminate whether the respective balloons 30 and 37 are in an inflated state or in a deflated state.

Fig. 3 shows an example of the remote controller 15, in which a shape of the remote controller 15, a shape and arrangement of each button (switch), and a function of each button (switch) can be appropriately changed.

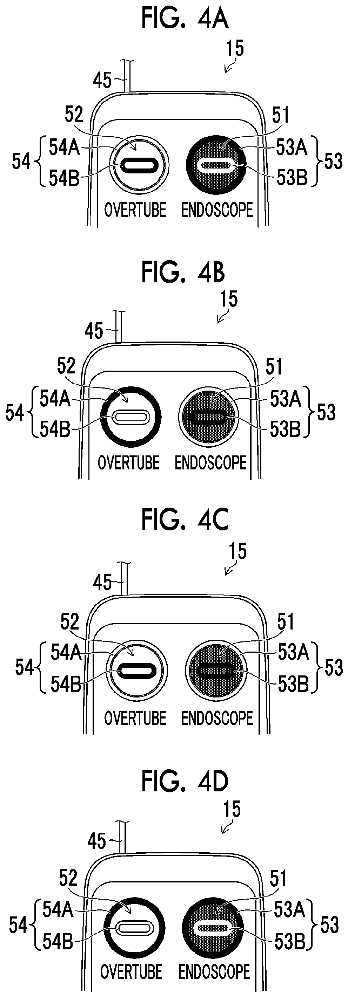

Figs. 4A to 4D are explanatory diagrams for describing a display pattern of the first balloon state display part 53 and the second balloon state display part 54 of the remote controller 15. For example, as shown in Fig. 4A, in a case in which the inflated state display part 53A is in a non-display state, the deflated state display part 53B is in a display state, the inflated state display part 54A is in a display state, and the deflated state display part 54B is in a non-display state, the operator can discriminate that the first balloon 30 is in a deflated state and the second balloon 37 is in an inflated state.

In addition, as shown in Fig. 4B, in a case in which the inflated state display part 53A is in a display state, the deflated state display part 53B is in a non-display state, the inflated state display part 54A is in a non-display state, and the deflated state display part 54B is in a display state, the operator can discriminate that the first balloon 30 is in an inflated state and the second balloon 37 is in a deflated state.

Further, as shown in Fig. 4C, in a case in which both of the inflated state display parts 53A and 54A are in a display state and both of the deflated state display parts 53B and 54B are in a non-display state, the operator can discriminate that both the balloons 30 and 37 are in an inflated state. Furthermore, as shown in Fig. 4D, in a case in which both of the inflated state display parts 53A and 54A are in a non-display state and both of the deflated state display parts 53B and 54B are in a display state, the operator can discriminate that both the balloons 30 and 37 are in a deflated state.

Returning to Fig. 3, a character string of "ENDOSCOPE" meaning the endoscope 10 is attached below the first balloon operating part 51, and a character string of "OVERTUBE" meaning the overtube 11 is below the second balloon operating part 52. Note that the present invention is not limited to this, and illustrations or the like showing the endoscope 10 and the overtube 11 may be attached.

The first balloon pause button 55 is provided below the character string of "ENDOSCOPE", and the second balloon pause button 56 is provided below the character string of "OVERTUBE". The first balloon pause button 55 and the second balloon pause button 56 are rectangular push buttons. A character string of "PAUSE" meaning a pause is attached between the first balloon pause button 55 and the second balloon pause button 56.

The first balloon pause button 55 inputs a pause operation of an inflated state and a deflated state of the first balloon 30 to the balloon controller 14. The balloon controller 14 that has received the input of the pause operation maintains the first balloon 30 in an inflated state by maintaining the pressure of the air supplied to the first balloon 30 at a constant value, or maintains the first balloon 30 in a deflated state by maintaining the pressure of the air sucked from the first balloon 30 at a constant value.

The second balloon pause button 56 inputs a pause operation of an inflated state and a deflated state of the second balloon 37 to the balloon controller 14. The balloon controller 14 that has received the input of the pause operation maintains the second balloon 37 in an inflated state by maintaining the pressure of the air supplied to the second balloon 37 at a constant value, or maintains the second balloon 37 in a deflated state by maintaining the pressure of the air sucked from the second balloon 37 at a constant value.

The stop button 57 is provided below the first balloon pause button 55 and the second balloon pause button 56 and on the center line of the remote controller 15. The stop button 57 is a rectangular push button. A character string of "STOP" meaning a stop is attached above the stop button 57.

The stop button 57 outputs a stop operation of the aforementioned buzzer sound (including an error code display) to the balloon controller 14. The balloon controller 14 stops the output of the buzzer sound in a case in which the stop operation is input while the buzzer sound is being output. In addition, instead of using the stop button 57 for the stop operation of the buzzer sound, the stop button 57 may be used for the aforementioned emergency stop of the inflation and deflation operation of the respective balloons 30 and 37.

Fig. 5 is an explanatory diagram for describing assignment of functions of some switches of the remote controller 15 to the multifunctional switches 29. Reference numerals 5A and 5B in Fig. 5 are enlarged views of the operating part 18 viewed from different directions, and reference numeral 5C is a front view of the remote controller 15.

As shown in Fig. 5, the operating part 18 is provided with five (or other than five) multifunctional switches 29 (hereinafter, referred to as multifunctional switches 29a to 29e as appropriate). The multifunctional switch 29 can input a plurality of types of balloon operations common to the remote controller 15. In the present embodiment, in a simulating manner, a function of the first balloon operating part 51 is assigned to the multifunctional switch 29e, and a function of the second balloon operating part 52 is assigned to the multifunctional switch 29d. Accordingly, the multifunctional switch 29e functions as a toggle switch similar to the aforementioned first balloon operating part 51, and the multifunctional switch 29d functions as a toggle switch similar to the aforementioned second balloon operating part 52.

Therefore, each time the multifunctional switch 29e is pushed, the inflation operation of the first balloon 30 and the deflation operation of the first balloon 30 are alternately input to the balloon controller 14 via a signal cable (not shown). In addition, each time the multifunctional switch 29d is pushed, the inflation operation of the second balloon 37 and the deflation operation of the second balloon 37 are alternately input to the balloon controller 14 via a signal cable (not shown). Accordingly, by pushing the multifunctional switches 29e and 29d, the respective balloons 30 and 37 can be individually switched between the inflation state and the deflation state. Operation information of the multifunctional switch 29e (inflation operation and deflation operation of the first balloon 30) and operation information of the multifunctional switch 29d (inflation operation and deflation operation of the second balloon 37) are alto sequentially input to the processor device 13 via a signal cable (not shown).

A function of the stop button 57 may be assigned to the multifunctional switch 29c. In addition, a function as an imaging button for executing the capturing of an image of the area to be observed by the imaging unit 24a (see Fig. 6) is assigned to the multifunctional switch 29a. In addition, a function as a changeover switch for switching observation modes such as normal observation and special light observation is assigned to the multifunctional switch 29b.

Returning to Fig. 1, the processor device 13 causes the monitor 16 to display an endoscopy screen 100 including an endoscope image 70 (see Fig. 6) of the area to be observed based on the imaging signal of the observed part sequentially input from the imaging unit 24a (see Fig. 6) via a signal cable (not shown) and the processor connector 22.

The processor device 13 comprises an arithmetic circuit configured of various processors, a memory, and the like. Various processors include a central processing unit (CPU), a graphics processing unit (GPU), an application specific integrated circuit (ASIC), and a programmable logic device [for example, simple programmable logic devices (SPLD), complex programmable logic device (CPLD), and field programmable gate arrays (FPGA)]. Note that various functions of the processor device 13 may be realized by one processor or may be realized by a plurality of processors of the same type or different types.

The processor device 13 has a normal mode and a balloon operation presentation mode as a display mode for displaying the endoscopy screen 100 on the monitor 16. The normal mode is a display mode selected in a case in which the remote controller 15 is used for the input of the balloon operations (inflation operation, deflation operation, and the like) of the respective balloons 30 and 37. Since the normal mode is the same as the mode in the related art, the description thereof will be omitted here.

The balloon operation presentation mode is a display mode selected in a case in which the multifunctional switch 29 (here, in particular, the multifunctional switches 29e and 29d) is used for the input of the balloon operations of the respective balloons 30 and 37. As described above, in a case in which the inflation operation and the deflation operation of the first balloon 30 are performed by using the multifunctional switch 29e, or the inflation operation and the deflation operation of the second balloon 37 are performed by using the multifunctional switch 29d, it is difficult to instantly grasp which balloon operation is being performed because the operator gazes at the monitor 16. Therefore, in a case in which the balloon operation presentation mode is selected, the processor device 13 causes the monitor 16 to display, together with the endoscope image 70, a remote controller image 72 in which the operator can easily identify the type of the balloon operation input to the multifunctional switch 29.

Fig. 6 is an explanatory diagram showing an example of the endoscopy screen 100 (the endoscope image 70 and the remote controller image 72) displayed on the monitor 16 in a balloon operation presentation mode by the processor device 13. In Fig. 6, a case in which only the multifunctional switches 29e and 29d are used for the input of the balloon operation will be described as an example. As shown in Fig. 6, in a balloon operation presentation mode, a processor 13a of the processor device 13 functions as a display control unit 80 by executing a program read out from a memory (not shown).

The display control unit 80 sequentially generates the endoscopy screen 100 and sequentially outputs it to the monitor 16 based on the imaging signal sequentially input from the imaging unit 24a via a signal cable (not shown) and the operation information of the multifunctional switches 29e and 29d sequentially input from the operating part 18 via a signal cable (not shown).

The endoscopy screen 100 is broadly divided into a first display region 100a and a second display region 100b. The first display region 100a displays the endoscope image 70 of a known area to be observed, which is generated by the display control unit 80 based on the imaging signal. The second display region 100b displays the remote controller image 72 generated by the display control unit 80 based on the operation information of the multifunctional switches 29e and 29d.

**In** the present embodiment, the endoscope image 70 and the remote controller image 72 are displayed in the first display region 100a and the second display region 100b, which are different from each other, in the endoscopy screen 100, but for example, the remote controller image 72 may be superimposed (superposed) on the endoscope image 70. Alternatively, the remote controller image 72 may be displayed in a display window different from a display window of the endoscope image 70 by a picture-in-picture method.

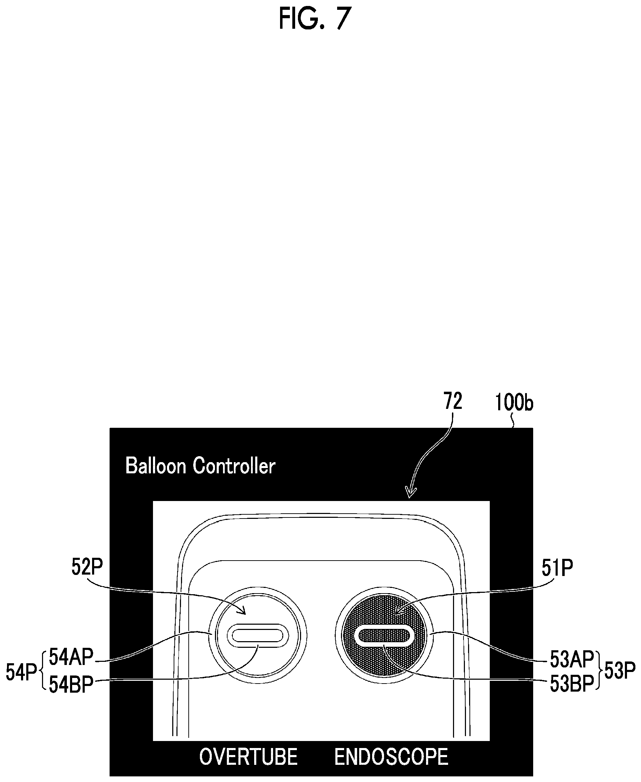

Fig. 7 is an enlarged view of the remote controller image 72 in the second display region 100b. As shown in Fig. 7, the remote controller image 72 is an image showing the remote controller 15. Here, the remote controller image 72 may be an entire image of the remote controller 15, but may be a partial image of the remote controller 15 as in the present embodiment. Specifically, the remote controller image 72 may be a partial image of the remote controller 15 in a case in which the remote controller image 72 includes an image of an individual operation switch of the remote controller 15 corresponding to the multifunctional switch 29 to which the balloon operation is assigned, here, images of the respective balloon operating parts 51 and 52 corresponding to the multifunctional switches 29e and 29d.

The remote controller image 72 includes at least a first balloon operating part image 51P corresponding to the first balloon operating part 51 and a second balloon operating part image 52P corresponding to the second balloon operating part 52. Here, in the present embodiment, the first balloon operating part 51 is provided with the first balloon state display part 53, and the second balloon operating part 52 is provided with the second balloon state display part 54. Therefore, the remote controller image 72 includes a first balloon state display part image 53P corresponding to the first balloon state display part 53, and a second balloon state display part image 54P corresponding to the second balloon state display part 54.

The first balloon state display part image 53P includes an inflated state display part image 53AP corresponding to the inflated state display part 53A and a deflated state display part image 53BP corresponding to the deflated state display part 53B. In addition, the second balloon state display part image 54P includes an inflated state display part image 54AP corresponding to the inflated state display part 54A and a deflated state display part image 54BP corresponding to the deflated state display part 54B.

In the remote controller image 72, similarly to the remote controller 15, a character string or an illustration indicating the endoscope 10 (that is, the first balloon 30) is displayed at a position near the first balloon operating part image 51P, and a character string or an illustration indicating the overtube 11 is displayed at a position near the second balloon operating part image 52P.

Each time the inflation operation or the deflation operation is input to the multifunctional switches 29e and 29d, the display control unit 80 reflects the type of the input operation on the remote controller image 72. For example, the display control unit 80 changes a display mode of the inflated state display part image 53AP in a case in which the inflation operation is performed with respect to the multifunctional switch 29e, and changes a display mode of the deflated state display part image 53BP in a case in which the deflation operation is performed with respect to the multifunctional switch 29e, based on the operation information input from the operating part 18.

In addition, for example, the display control unit 80 changes a display mode of the inflated state display part image 54AP in a case in which the inflation operation is performed with respect to the multifunctional switch 29d, and changes a display mode of the deflated state display part image 54BP in a case in which the deflation operation is performed with respect to the multifunctional switch 29d, based on the operation information input from the operating part 18.

The change in the display modes of the inflated state display part images 53AP and 54AP and the deflated state display part images 53BP and 54BP referred to here means, for example, blinking individual images or changing a color, shape, or size of the individual images.

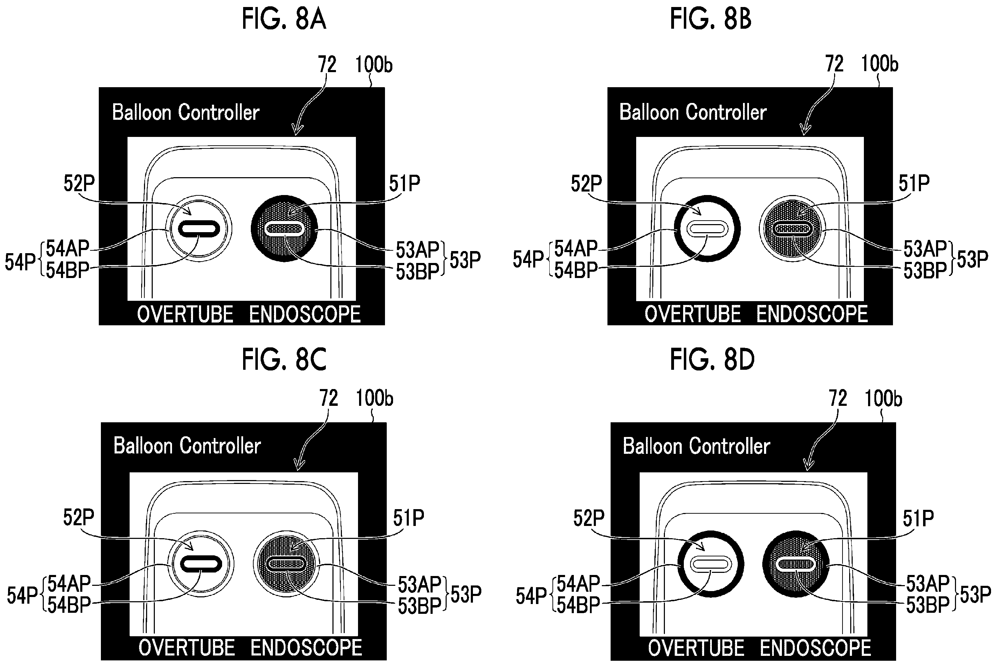

Figs. 8A to 8D are explanatory diagrams for describing an example of changing the display modes of the inflated state display part images 53AP and 54AP and the deflated state display part images 53BP and 54BP in accordance with the inflation operation or the deflation operation with respect to the multifunctional switches 29e and 29d.

As shown in Fig. 8A, the display control unit 80 changes the display modes of the deflated state display part image 53BP (the first balloon operating part image 51P) and the inflated state display part image 54AP (the second balloon operating part image 52P) in accordance with the deflation operation of the first balloon 30 with respect to the multifunctional switch 29e and the inflation operation of the second balloon 37 with respect to the multifunctional switch 29d.

In addition, as shown in Fig. 8B, the display control unit 80 changes the display modes of the inflated state display part image 53AP (the first balloon operating part image 51P) and the deflated state display part image 54BP (the second balloon operating part image 52P) in accordance with the inflation operation of the first balloon 30 with respect to the multifunctional switch 29e and the deflation operation of the second balloon 37 with respect to the multifunctional switch 29d.

Further, as shown in Fig. 8C, the display control unit 80 changes the display modes of the inflated state display part images 53AP and 54AP (the first balloon operating part image 51P and the second balloon operating part image 52P) in accordance with the inflation operation of the respective balloons 30 and 37 with respect to the multifunctional switches 29e and 29d. Furthermore, as shown in Fig. 8D, the display control unit 80 changes the display modes of the deflated state display part images 53BP and 54BP (the first balloon operating part image 51P and the second balloon operating part image 52P) in accordance with the deflation operation of the respective balloons 30 and 37 with respect to the multifunctional switches 29e and 29d.

Hereinafter, each time the operator inputs the inflation operation or the deflation operation of the respective balloons 30 and 37 with respect to the multifunctional switches 29e and 29d, the change in the display modes of the first balloon operating part image 51P (the inflated state display part image 53AP or the deflated state display part image 53BP) and the second balloon operating part image 52P (the inflated state display part image 54AP or the deflated state display part image 54BP) by the display control unit 80 is repeatedly executed.

As described above, in the first embodiment, the change in the display mode of the first balloon operating part image 51P corresponding to the inflation operation and the deflation operation of the first balloon 30 with respect to the multifunctional switch 29e and the change in the display mode of the second balloon operating part image 52P corresponding to the inflation operation and the deflation operation of the second balloon 37 with respect to the multifunctional switch 29d are executed. As a result, the inflation operation and the deflation operation of the first balloon 30 with respect to the multifunctional switch 29e are individually reflected on the remote controller image 72, and the inflation operation and the deflation operation of the second balloon 37 with respect to the multifunctional switch 29d are individually reflected on the remote controller image 72. Accordingly, the operator can easily grasp what kind of operation has been performed on the multifunctional switches 29e and 29d in a state in which the operator gazes at the monitor 16.

In addition, in the present embodiment, the operation with respect to the multifunctional switches 29e and 29d is reflected on the remote controller image 72 of the remote controller 15 used for the inflation operation and the deflation operation of the respective balloons 30 and 37. Accordingly, the operator can more intuitively grasp which switch of the remote controller 15 the operation with respect to the multifunctional switches 29e and 29d corresponds to, as compared with the case in which only the character string or the illustration indicating the inflation operation and the deflation operation of the respective balloons 30 and 37 is displayed on the monitor 16.

### Second Embodiment

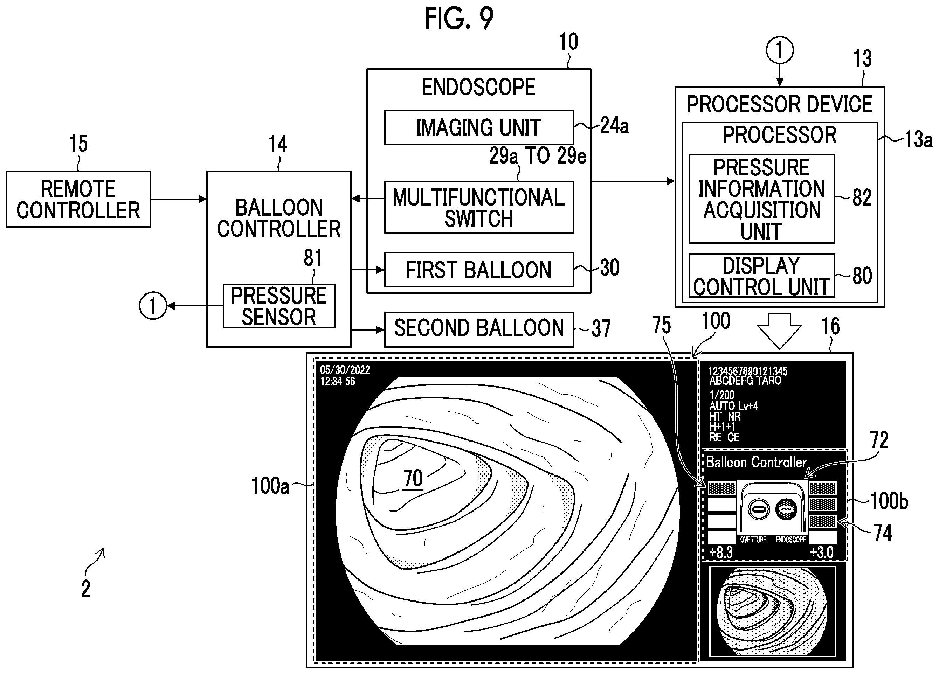

Fig. 9 is an explanatory diagram showing an example of the endoscopy screen 100 displayed on the monitor 16 by the processor device 13 of the endoscope device 2 according to a second embodiment. The endoscope device 2 of the second embodiment displays information indicating pressure in the first balloon 30 and pressure in the second balloon 37 on the endoscopy screen 100.

As shown in Fig. 9, the endoscope device 2 according to the second embodiment has basically the same configuration as the endoscope device 2 according to the first embodiment except that the balloon controller 14 is provided with a pressure sensor 81 and the processor 13a of the processor device 13 functions as a pressure information acquisition unit 82. Therefore, the same reference numerals are given to those having the same function or configuration as those of the first embodiment, and the description thereof will be omitted.

The pressure sensor 81 corresponds to a pressure detection sensor of the present invention, and is provided corresponding to the respective balloons 30 and 37 in the balloon controller 14. The pressure sensor 81 repeatedly executes detection of the pressure in the first balloon 30 and detection of the pressure in the second balloon 37 while the balloon controller 14 is being operated. Since a method of detecting the pressure in the respective balloons 30 and 37 by the pressure sensor 81 is a known technology (for example, see JP2005-261781A above), specific description thereof will be omitted here. The pressure sensor 81 may be provided in the endoscope 10 instead of being provided in the balloon controller 14. Then, each time the pressure sensor 81 detects the pressure in the respective balloons 30 and 37, a pressure detection result of the respective balloons 30 and 37 is repeatedly output to the processor device 13 via a communication cable (not shown).

The pressure information acquisition unit 82 repeatedly executes acquisition of the pressure detection results of the respective balloons 30 and 37 from the pressure sensor 81 and output of the pressure detection results of the respective balloons 30 and 37 to the display control unit 80.

The display control unit 80 according to the second embodiment sequentially generates the endoscopy screen 100 and sequentially outputs it to the monitor 16 based on the imaging signal sequentially input from the imaging unit 24a, the operation information of the multifunctional switches 29e and 29d sequentially input from the operating part 18, and the output of the pressure detection results of the respective balloons 30 and 37 input from the pressure information acquisition unit 82.

The endoscopy screen 100 according to the second embodiment is basically the same as the endoscopy screen 100 according to the first embodiment except that the second display region 100b includes, in addition to the remote controller image 72, first pressure information 74 indicating the pressure in the first balloon 30 and the second pressure information 75 indicating the pressure in the second balloon 37.

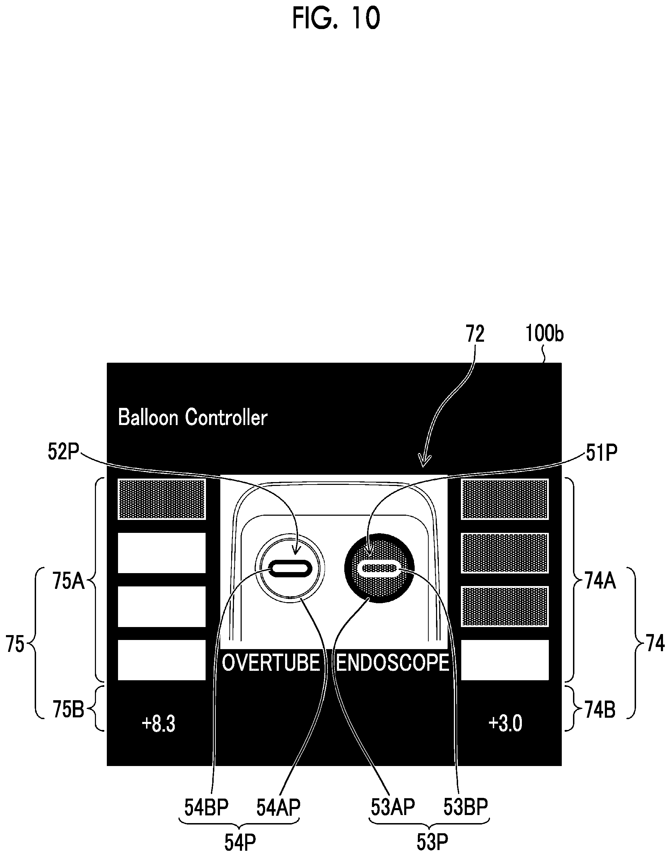

Fig. 10 is an enlarged view of the remote controller image 72, the first pressure information 74, and the second pressure information 75 according to the second embodiment. As shown in Fig. 10, the display control unit 80 generates the first pressure information 74 based on the pressure detection result of the first balloon 30 input from the pressure information acquisition unit 82, and displays the first pressure information 74 at a position near the first balloon operating part image 51P. In addition, the display control unit 80 generates the second pressure information 75 based on the pressure detection result of the second balloon 37 input from the pressure information acquisition unit 82, and displays the second pressure information 75 at a position near the second balloon operating part image 52P.

The first pressure information 74 includes an indicator 74Aindicating magnitude of the pressure in the first balloon 30, and a pressure numerical value 74B indicating a numerical value of the pressure in the first balloon 30. The second pressure information 75 includes an indicator 75A indicating magnitude of the pressure in the second balloon 37, and a pressure numerical value 75B indicating a numerical value of the pressure in the second balloon 37.

In the second embodiment, both the indicator 74A and the pressure numerical value 74B are displayed in the remote controller image 72 as the first pressure information 74, but only any one of the indicator 74A or the pressure numerical value 74B may be displayed. For example, by operating a display changeover switch (a function may be assigned to the multifunctional switch 29) (not shown) provided in the balloon controller 14, it is possible to switch between displaying both the indicator 74A and the pressure numerical value 74B, displaying only the indicator 74A, and displaying only the pressure numerical value 74B. Similarly, for the second pressure information 75, it may be possible to switch between displaying both the indicator 75A and the pressure numerical value 75B, displaying only the indicator 75A, and displaying only the pressure numerical value 75B.

Hereinafter, the display control unit 80 updates the display of the first pressure information 74 and the second pressure information 75 each time new pressure detection results of the respective balloons 30 and 37 are input from the pressure information acquisition unit 82.

As described above, in the second embodiment, by displaying the first pressure information 74 and the second pressure information 75 on the monitor 16, the operator can easily grasp the pressure in the respective balloons 30 and 37 in a state in which the operator gazes at the monitor 16. In addition, by displaying the first pressure information 74 at a position near the first balloon operating part image 51P and displaying the second pressure information 75 at a position near the second balloon operating part image 52P, the operator can easily distinguish between the pressure in the first balloon 30 and the pressure in the second balloon 37.

In the second embodiment, the first pressure information 74 and the second pressure information 75 are displayed at positions near the remote controller image 72, but the first pressure information 74 may be superimposed on the first balloon operating part image 51P, and the second pressure information 75 may be superimposed on the second balloon operating part image 52P. In addition, the first pressure information 74 and the second pressure information 75 may be displayed at any positions in the endoscopy screen 100.

### Third Embodiment

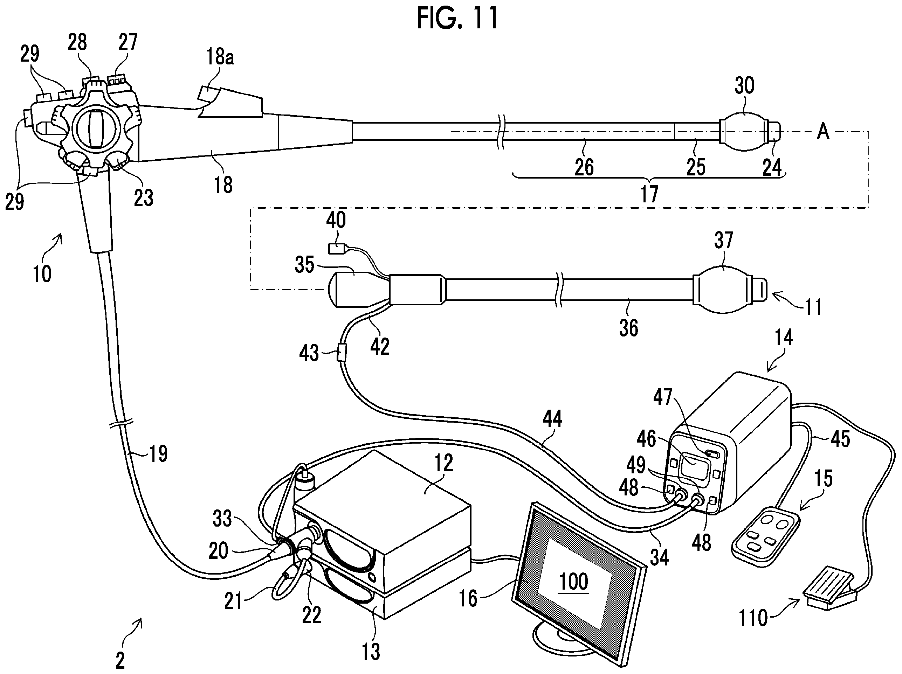

Fig. 11 is a configuration diagram of the endoscope device 2 according to a third embodiment. In each of the above-described embodiments, it is necessary to operate the emergency stop switch 48 provided on the front surface of the balloon controller 14 in a case of executing an emergency stop of the balloon controller 14 (an emergency stop of the inflation and deflation operation of the respective balloons 30 and 37), but the emergency stop operation may take time because both hands of the operator are full.

Therefore, in the endoscope device 2 according to the third embodiment, a foot switch 110 for emergency stop is connected to the balloon controller 14. The endoscope device 2 according to the third embodiment has basically the same configuration as the endoscope device 2 according to each of the above-described embodiments except that the foot switch 110 is provided and the functions of the display control unit 80 are partially different. Therefore, the same reference numerals are given to those having the same function or configuration as those of each of the above-described embodiments, and the description thereof will be omitted.

The foot switch 110 corresponds to the emergency stop switch of the present invention, and receives a foot operation (foot-step operation) by an operator's foot. The balloon controller 14 according to the third embodiment executes an emergency stop (a stop of the inflation and deflation operation of the respective balloons 30 and 37 or deflation of the respective balloons 30 and 37) in a case in which the foot operation is performed with respect to the foot switch 110. Two foot switches 110 may be connected to the balloon controller 14 so that the balloon controller 14 can individually execute the emergency stop of the respective balloons 30 and 37.

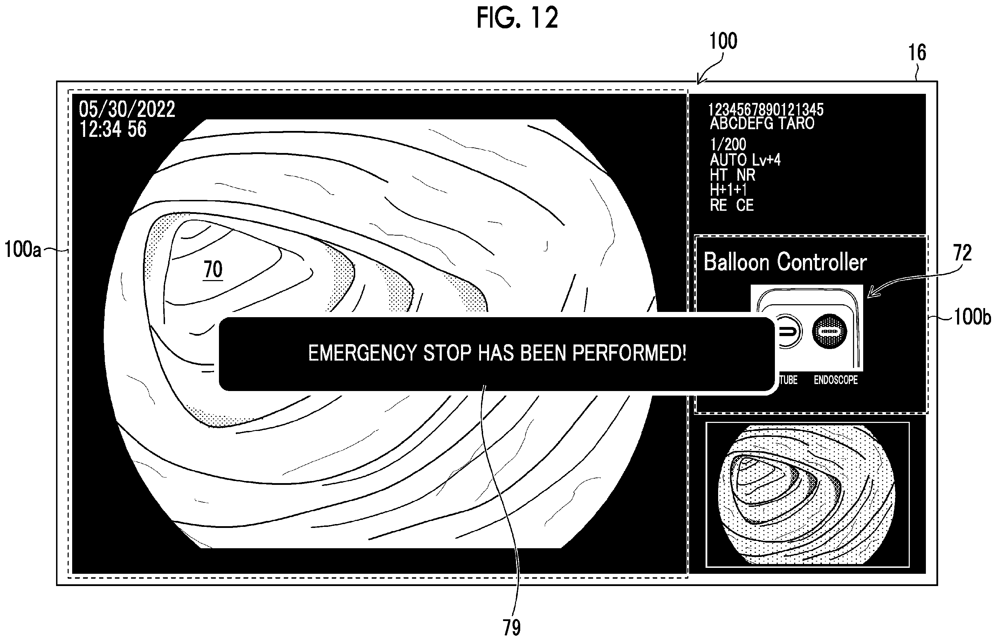

Fig. 12 is an explanatory diagram for describing a display of warning information 79 by the monitor 16. As shown in Fig. 12, the display control unit 80 according to the third embodiment causes the monitor 16 to display the warning information 79 indicating that the balloon controller 14 has been brought to an emergency stop in a case in which the foot operation is performed with respect to the foot switch 110, for example, superimposes the warning information 79 on the endoscopy screen 100. Accordingly, it is possible to notify that the balloon controller 14 has been brought to an emergency stop. In addition to or instead of displaying the warning information 79 on the monitor 16, the warning information 79 described above may be output as audio from a speaker (not shown).

As described above, in the third embodiment, since an emergency stop of the balloon controller 14 can be performed by the foot switch 110, the emergency stop of the balloon controller 14 can be quickly executed even in a case in which both hands of the operator are full.

In the third embodiment, the emergency stop of the balloon controller 14 is made possible by the foot switch 110, but for example, the function of the emergency stop switch may be provided in the remote controller 15 or the multifunctional switch 29.

### Others

In each of the above-described embodiments, as a method of reflecting the type of the balloon operation (inflation operation, deflation operation) input to the multifunctional switches 29e and 29d on the remote controller image 72, the change in the display modes of the first balloon operating part image 51P and the second balloon operating part image 52P is given as an example, the present invention is not limited thereto. As long as the operator can grasp the type of the balloon operation input to the multifunctional switch 29, the method of reflecting the type of the balloon operation on the remote controller image 72 is not particularly limited. For example, a character string or an illustration indicating the type of the balloon operation input to the multifunctional switch 29 may be displayed on the remote controller image 72 or at a position near the remote controller image 72.

In each of the above-described embodiments, the functions of the respective balloon operating parts 51 and 52 of the remote controller 15 are assigned to each multifunctional switch 29, but the functions of the stop button 57 as shown in Fig. 5 or the functions of other buttons and switches may be assigned to the multifunctional switches 29. In this case, the remote controller image 72 includes an image of the individual operation switch of the remote controller 15 corresponding to the multifunctional switch 29 to which the balloon operation is assigned.

In each of the above-described embodiments, the functions of some individual operation switches of the remote controller 15 are assigned to the multifunctional switches 29 provided in the operating part 18, but the controller operation switch of the present invention is not limited to the multifunctional switch 29. For example, the operating part 18 may be provided with dedicated operation switches having the functions of at least some individual operation switches of the remote controller 15. Further, the multifunctional switch 29 or the dedicated operation switch may be provided in addition to the operating part 18 of the endoscope 10.

In each of the above-described embodiments, the remote controller image 72, the first pressure information 74, and the second pressure information 75 are displayed on the endoscopy screen 100 only in the balloon operation presentation mode in which the multifunctional switch 29 is used, but the remote controller image 72, the first pressure information 74, and the second pressure information 75 may be displayed on the endoscopy screen 100 even in the normal mode. **In** this case, the operation on the remote controller 15 is reflected on the remote controller image 72.

Although the double-balloon type endoscope device 2 has been described in each of the above-described embodiments, the present invention can also be applied to a single-balloon type endoscope device 2 (endoscope 10).

### Explanation of References

2: endoscope device
10: endoscope
11: overtube
12: light source device
13: processor device
13a: processor
14: balloon controller
15: remote controller
16: monitor
17: insertion part
18: operating part
18a: treatment tool insertion part
19: universal cable
20: light source connector
21: cable
22: processor connector
23: angle knob
24: distal end portion
24a: imaging unit
25: bendable portion
26: soft portion
27: air supply/water supply button
28: suction button
29: multifunctional switch
29a, 29b, 29c, 29d, 29e: multifunctional switch
30: first balloon
31: first supply/discharge pipe line
32: opening
33: endoscope-side cap
34: tube
35: grip portion
36: main body portion
37: second balloon
38: supply pipe line
39: second supply/discharge pipe line
40: connector
41: opening
42: tube
43: connector
44: tube
45: cable
46: display part
47: power switch
48: emergency stop switch
49: tube connecting portion
51: first balloon operating part
51P: first balloon operating part image
52: second balloon operating part
52P: second balloon operating part image
53: first balloon state display part
53A: inflated state display part
53AP: inflated state display part image
53B: deflated state display part
53BP: deflated state display part image
53P: first balloon state display part image
54: second balloon state display part
54A: inflated state display part
54AP: inflated state display part image
54B: deflated state display part
54BP: deflated state display part image
54P: second balloon state display part image
55: first balloon pause button
56: second balloon pause button
57: stop button
70: endoscope image
72: remote controller image
74: first pressure information
74A: indicator
74B: pressure numerical value
75: second pressure information
75A: indicator
75B: pressure numerical value
79: warning information
80: display control unit
81: pressure sensor
82: pressure information acquisition unit
100: endoscopy screen
100a: first display region
100b: second display region
110: foot switch

## Claims

1. An endoscope device (2) comprising:
a balloon (30,37);
a balloon controller (14) that controls inflation and deflation of the balloon (30,37);
a remote controller (15) that is connected to the balloon controller (14) and operates the balloon controller (14), the remote controller (15) being capable of inputting a plurality of types of balloon operations including an inflation operation and a deflation operation of the balloon (30,37);
a controller operation switch for operating the balloon controller (14), the controller operation switch being provided in an endoscope (10) and capable of inputting a plurality of types of the balloon (30,37) operations that are common to the remote controller (15); and
a processor (13a),
wherein the processor (13a) is configured to:
cause a monitor (16) to display an endoscope image (70) captured by the endoscope (10) and a remote controller image (72) which is an image of the remote controller (15); and
in a case in which the balloon (30,37) operation is input to the controller operation switch, reflect a type of the balloon operation input to the controller operation switch on the remote controller image (72) displayed on the monitor (16).

2. The endoscope device (2) according to claim 1,
wherein the remote controller (15) includes a plurality of types of individual operation switches corresponding to the plurality of types of balloon operations,
the remote controller image (72) includes images of the plurality of types of individual operation switches, and
the processor (13a) is configured to, in a case in which the balloon operation is input to the controller operation switch, change a display mode of an image of the individual operation switch corresponding to the type of the balloon operation.

3. The endoscope device (2) according to claim 1 or 2,
wherein the balloon (30,37) is provided at at least one of a distal end portion (24) of an insertion part of the endoscope or a distal end portion (24) of an overtube (11) through which the insertion part is inserted.

4. The endoscope device (2) according to claim 3,
wherein the balloon (30,37) is provided at the distal end portion (24) of the insertion part and the distal end portion (24) of the overtube (11),
the balloon controller (14) controls inflation and deflation of the balloon (30,37) for each of the balloons (30,37),
the remote controller (15) is capable of inputting the balloon operation for each of the balloons (30,37), and
the processor (13a) is configured to reflect the type of the balloon operation input to the controller operation switch on the remote controller image (72), for each of the balloons (30,37).

5. The endoscope device (2) according to any one of claims 1 to 4, further comprising:
an emergency stop switch (48) for bringing the balloon controller (14) to an emergency stop,
wherein the processor (13a) is configured to, in a case in which the emergency stop switch (48) is operated, cause the monitor (16) to display information indicating that the balloon controller (14) has been brought to an emergency stop.

6. The endoscope device (2) according to claim 5,
wherein the emergency stop switch (48) is a foot switch (110) connected to the balloon controller (14).

7. The endoscope device (2) according to any one of claims 1 to 6, further comprising:
a pressure detection sensor (81) that repeatedly detects a pressure inside the balloon (30,37),
wherein the processor (13a) is configured to cause the monitor (16) to display information indicating the pressure each time the pressure detection sensor (81) detects the pressure.

8. The endoscope device (2) according to claim 7,
wherein the balloon is provided at a distal end portion (24) of an insertion part of the endoscope (10) and a distal end portion (24) of an overtube (11) through which the insertion part is inserted,
the pressure detection sensor (81) repeatedly detects the pressure for each of the balloons (30,37), and
the processor (13a) is configured to cause the monitor (16) to display the information indicating the pressure for each of the balloons (30,37).

9. The endoscope device (2) according to any one of claims 1 to 8,
wherein the controller operation switch is provided in an operating part (18) provided on a base end side of an insertion part of the endoscope (10).

10. The endoscope device (2) according to claim 9,
wherein the remote controller (15) includes a plurality of types of individual operation switches corresponding to the plurality of types of balloon operations,
the operating part (18) is provided with a plurality of multifunctional switches to which a plurality of types of operations are capable of being selectively assigned, and
the controller operation switch is the plurality of multifunctional switches to which functions of the plurality of types of individual operation switches are individually assigned.

## Patentansprüche

1. Endoskopvorrichtung (2), umfassend:
einen Ballon (30, 37);
eine Ballonsteuerung (14), die Inflation und Deflation des Ballons (30, 37) steuert;
eine Fernsteuerung (15), die mit der Ballonsteuerung (14) verbunden ist und die Ballonsteuerung (14) betreibt, wobei die Fernsteuerung (15) in der Lage ist, mehrere Typen von Bedienungen von Ballon einschließlich einer Inflationsbedienung und einer Deflationsbedienung des Ballons (30, 37) einzugeben;
einen Steuerungsbedienungsschalter zum Bedienen der Ballonsteuerung (14), wobei der Steuerungsbedienungsschalter in einem Endoskop (10) vorgesehen ist und in der Lage ist, mehrere Typen der Bedienungen von Ballon (30, 37), die der Fernsteuerung (15) gemeinsam sind, einzugeben; und
einen Prozessor (13a),
wobei der Prozessor (13a) so konfiguriert ist, dass er:
einen Monitor (16) veranlasst, ein Endoskopiebild (70), das von dem Endoskop (10) aufgenommen wird, und ein Fernsteuerungsbild (72), das ein Bild der Fernsteuerung (15) ist, anzuzeigen; und
in einem Fall, in dem die Bedienung (30, 37) von Ballon in den Steuerungsbedienungsschalter für eingegeben wird, einen Typ der Bedienung von Ballon, die in den Steuerungsbedienungsschalter eingegeben wird, auf dem Fernsteuerungsbild (72), das auf dem Monitor (16) angezeigt wird, widerspiegelt.

2. Endoskopvorrichtung (2) nach Anspruch 1,
wobei die Fernsteuerung (15) mehrere Typen von einzelnen Bedienungsschaltern, die den mehreren Typen von Bedienungen von Ballon entsprechen, enthält,
das Fernsteuerungsbild (72) Bilder der mehreren Typen von einzelnen Bedienungsschaltern enthält, und
der Prozessor (13a) so konfiguriert ist, dass er in einem Fall, in dem die Bedienung von Ballon in den Steuerungsbedienungsschalter eingegeben wird, einen Anzeigemodus eines Bildes des einzelnen Bedienungsschalters, der dem Typ der Bedienung von Ballon entspricht, ändert.

3. Endoskopvorrichtung (2) nach Anspruch 1 oder 2,
wobei der Ballon (30, 37) an mindestens einem von einem distalen Endabschnitt (24) eines Einführteils des Endoskops und einem distalen Endabschnitt (24) eines Überrohrs (11), durch das der Einführteil eingeführt wird, vorgesehen ist.

4. Endoskopvorrichtung (2) nach Anspruch 3,
wobei der Ballon (30, 37) an dem distalen Endabschnitt (24) des Einführteils und dem distalen Endabschnitt (24) des Überrohrs (11) vorgesehen ist,
die Ballonsteuerung (14) Inflation und Deflation des Ballons (30, 37) für jeden der Ballons (30, 37) steuert,
die Fernsteuerung (15) in der Lage ist, die Bedienung von Ballon für jeden der Ballons (30, 37) einzugeben, und
der Prozessor (13a) so konfiguriert ist, dass er den Typ der Bedienung von Ballon, die in den Steuerungsbedienungsschalter eingegeben wird, auf dem Fernsteuerungsbild (72) für jeden der Ballons (30, 37) widerspiegelt.

5. Endoskopvorrichtung (2) nach einem der Ansprüche 1 bis 4, ferner umfassend:
einen Not-Aus-Schalter (48) zum Bringen der Ballonsteuerung (14) in einen Not-Aus-Zustand,
wobei der Prozessor (13a) so konfiguriert ist, dass er in einem Fall, in dem der Not-Aus-Schalter (48) betätigt wird, den Monitor (16) veranlasst, Informationen anzuzeigen, die angeben, dass die Ballonsteuerung (14) in einen Not-Aus-Zustand gebracht wurde.

6. Endoskopvorrichtung (2) nach Anspruch 5,
wobei der Not-Aus-Schalter (48) ein Fußschalter (110) ist, der mit der Ballonsteuerung (14) verbunden ist.

7. Endoskopvorrichtung (2) nach einem der Ansprüche 1 bis 6, ferner umfassend:
einen Druckdetektionssensor (81), der wiederholt einen Druck innerhalb des Ballons (30, 37) detektiert,
wobei der Prozessor (13a) so konfiguriert ist, dass er den Monitor (16) veranlasst, Informationen anzuzeigen, die den Druck angeben, jedes Mal, wenn der Druckdetektionssensor (81) den Druck detektiert.

8. Endoskopvorrichtung (2) nach Anspruch 7,
wobei der Ballon an einem distalen Endabschnitt (24) eines Einführteils des Endoskops (10) und einem distalen Endabschnitt (24) eines Überrohrs (11), durch das der Einführteil eingeführt wird, vorgesehen ist,
der Druckdetektionssensor (81) den Druck für jeden der Ballons (30, 37) wiederholt detektiert, und
der Prozessor (13a) so konfiguriert ist, dass er den Monitor (16) veranlasst, die Informationen, die den Druck angeben, für jeden der Ballons (30, 37) anzuzeigen.

9. Endoskopvorrichtung (2) nach einem der Ansprüche 1 bis 8;
wobei der Steuerungsbedienungsschalter in einem Bedienungsteil (18), der an einer Basisendseite eines Einführteils des Endoskops (10) vorgesehen ist, vorgesehen ist.

10. Endoskopvorrichtung (2) nach Anspruch 9,
wobei die Fernsteuerung (15) mehrere Typen von einzelnen Bedienungsschaltern, die den mehreren Typen von Bedienungen von Ballon entsprechen, enthält,
der Bedienungsteil (18) mit mehreren multifunktionalen Schaltern versehen ist, denen mehrere Typen von Bedienungen selektiv zugewiesen werden können, und
der Steuerungsbedienungsschalter die mehreren multifunktionalen Schalter sind, denen Funktionen der mehreren Typen von einzelnen Bedienungsschaltern einzeln zuwiesen sind.

## Revendications

1. Dispositif endoscopique (2) comprenant :
un ballon (30, 37) ;
un contrôleur de ballon (14) qui contrôle l'inflation et la déflation du ballon (30, 37) ;
un contrôleur à distance (15) qui est connecté au contrôleur de ballon (14) et actionne le contrôleur de ballon (14), le contrôleur à distance (15) étant capable d'entrer une pluralité de types d'opérations de ballon incluant une opération d'inflation et une opération de déflation du ballon (30, 37) ;
un interrupteur d'opération de contrôleur pour actionner le contrôleur de ballon (14), l'interrupteur d'opération de contrôleur étant prévu dans un endoscope (10) et capable d'entrer une pluralité de types des opérations de ballon (30, 37) qui sont communes au contrôleur à distance (15) ; et
un processeur (13a),
dans lequel le processeur (13a) est configuré pour :
amener un moniteur (16) à afficher une image d'endoscope (70) capturée par l'endoscope (10) et une image de contrôleur à distance (72) qui est une image du contrôleur à distance (15) ; et
dans un cas où l'opération de ballon (30, 37) est entrée dans l'interrupteur d'opération de contrôleur, refléter un type de l'opération de ballon entrée dans l'interrupteur d'opération de contrôleur sur l'image de contrôleur à distance (72) affichée sur le moniteur (16).

2. Dispositif d'endoscope (2) selon la revendication 1,
dans lequel le contrôleur à distance (15) inclut une pluralité de types d'interrupteurs d'opération individuels correspondant à la pluralité de types d'opérations de ballon,
l'image de contrôleur à distance (72) inclut des images de la pluralité de types d'interrupteurs d'opération individuels, et
le processeur (13a) est configuré pour, dans un cas où l'opération de ballon est entrée dans l'interrupteur d'opération de contrôleur, changer un mode d'affichage d'une image de l'interrupteur d'opération individuel correspondant au type de l'opération de ballon.

3. Dispositif d'endoscope (2) selon la revendication 1 ou la revendication 2, dans lequel le ballon (30, 37) est prévu à au moins l'une d'une partie d'extrémité distale (24) d'une partie d'insertion de l'endoscope et d'une partie d'extrémité distale (24) d'un surtube (11) à travers lequel la partie d'insertion est insérée.

4. Dispositif d'endoscope (2) selon la revendication 3,
dans lequel le ballon (30, 37) est prévu à la partie d'extrémité distale (24) de la partie d'insertion et à la partie d'extrémité distale (24) du surtube (11),
le contrôleur de ballon (14) contrôle l'inflation et la déflation du ballon (30, 37) pour chacun des ballons (30, 37),
le contrôleur à distance (15) est capable d'entrer l'opération de ballon pour chacun des ballons (30, 37), et
le processeur (13a) est configuré pour refléter le type de l'opération de ballon entrée dans l'interrupteur d'opération de contrôleur sur l'image de contrôleur à distance (72), pour chacun des ballons (30, 37).

5. Dispositif endoscopique (2) selon l'une quelconque des revendications 1 à 4, comprenant en outre :
un interrupteur d'arrêt d'urgence (48) pour amener le contrôleur de ballon (14) à un arrêt d'urgence,
dans lequel le processeur (13a) est configuré pour, dans un cas où l'interrupteur d'arrêt d'urgence (48) est actionné, amener le moniteur (16) à afficher des informations indiquant que le contrôleur de ballon (14) a été amené à un arrêt d'urgence.

6. Dispositif d'endoscope (2) selon la revendication 5,
dans lequel l'interrupteur d'arrêt d'urgence (48) est un interrupteur de pédale (110) connecté au contrôleur de ballon (14).

7. Dispositif endoscopique (2) selon l'une quelconque des revendications 1 à 6, comprenant en outre :
un capteur de détection de pression (81) qui détecte de manière répétée une pression dedans du ballon (30, 37),
dans lequel le processeur (13a) est configuré pour amener le moniteur (16) à afficher des informations indiquant la pression chaque fois que le capteur de détection de pression (81) détecte la pression.

8. Dispositif d'endoscope (2) selon la revendication 7,
dans lequel le ballon est prévu à une partie d'extrémité distale (24) d'une partie d'insertion de l'endoscope (10) et à une partie d'extrémité distale (24) d'un surtube (11) à travers lequel la partie d'insertion est insérée,
le capteur de détection de pression (81) détecte de manière répétée la pression pour chacun des ballons (30, 37), et
le processeur (13a) est configuré pour amener le moniteur (16) à afficher les informations indiquant la pression pour chacun des ballons (30, 37).

9. Dispositif endoscopique (2) selon l'une quelconque des revendications 1 à 8 ;
dans lequel l'interrupteur d'opération de contrôleur est prévu dans une partie d'opération (18) prévue sur un côté d'extrémité de base d'une partie d'insertion de l'endoscope (10).

10. Dispositif d'endoscope (2) selon la revendication 9,
dans lequel le contrôleur à distance (15) inclut une pluralité de types d'interrupteurs d'opération individuels correspondant à la pluralité de types d'opérations de ballon,
la partie d'opération (18) est pourvue d'une pluralité d'interrupteurs multifonctionnels auxquels une pluralité de types d'opérations sont capables d'être attribuées sélectivement, et
l'interrupteur d'opération de contrôleur est la pluralité d'interrupteurs multifonctionnels auxquels des fonctions de la pluralité de types d'interrupteurs d'opération individuels sont attribuées individuellement.
